(19) Europäisches Patentamt
European Patent Office
Office européen des brevets

(11) Veröffentlichungsnummer: **0 310 854 B1**

(12) # EUROPÄISCHE PATENTSCHRIFT

(45) Veröffentlichungstag der Patentschrift: **20.05.92**

(51) Int. Cl.$^5$: **C07D 207/14**

(21) Anmeldenummer: **88115370.4**

(22) Anmeldetag: **20.09.88**

Die Akte enthält technische Angaben, die nach dem Eingang der Anmeldung eingereicht wurden und die nicht in dieser Patentschrift enthalten sind.

(54) **Verfahren zur Herstellung von 3-Amino-1-benzyl-pyrrolidinen.**

(30) Priorität: **02.10.87 DE 3733289**

(43) Veröffentlichungstag der Anmeldung: **12.04.89 Patentblatt 89/15**

(45) Bekanntmachung des Hinweises auf die Patenterteilung: **20.05.92 Patentblatt 92/21**

(84) Benannte Vertragsstaaten: **AT BE CH DE ES FR GB IT LI NL SE**

(56) Entgegenhaltungen:
**AT-B- 267 513**

**Revue Roumaine de Chemie, 15, 253 (1970)**

**JOURNAL OF MEDICINAL CHEMISTRY, Band 10, Dezember 1967; WILLIAM J. WELSTEAD et al.: "Aminoalkylindoles with Central Nervous System Activity" Seiten 1015-1021**

**JOURNAL OF MEDICINAL CHEMISTRY, Band 20, Nr. 6, 1977; DONALD T. WITIAK et al.: "3,4-Methylenedioxphenyl-, Isopropylidenedioxyphenyl, and Benzyl-Substituted Chiral 2-Aminosuccinicmides and 3-Aminopyrrolidines. Stereoselective Investigations of Potential Anti-Parkinsonian, Antipsychotic, and Anticonvulsant Activities" Seiten 801-805**

**JOURNAL OF MEDICINAL CHEMISTRY, Band 11, 1968; GROVER C. HELSLEY et al.: "Synthesis and Biological Activity of Some 1-Substituted 3-Pyrrolidinylureas" Seiten 1034-1037**

(73) Patentinhaber: **BAYER AG**

**W-5090 Leverkusen 1 Bayerwerk(DE)**

(72) Erfinder: **Krebs, Andreas, Dr.**
**Am Gartenfeld 70**
**W-5068 Odenthal-Holz(DE)**
Erfinder: **Schenke, Thomas, Dr.**
**Mühlenstrasse 113**
**W-5060 Bergisch-Gladbach 2(DE)**

**EP 0 310 854 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.08/2.19/2.0)

## Beschreibung

Die vorliegende Erfindung betrifft ein neues Verfahren zur Herstellung teilweise bekannter 3-Amino-1-benzyl-pyrrolidine, die in der 3- und/oder 4-Position sowie am Amin-Rest substituiert sein können.

Es ist bereits bekannt, daß 3-Arylaminopyrrolidine durch hydrogenolytische Debenzylierung entsprechender 3-Arylamino-1-benzylpyrrolidine erhalten werden können. Diese werden durch Umsetzung von Anilinen entweder mit 1-Benzyl-3-chlor- oder mit 1-Benzyl-3-tosyloxy-pyrrolidinen erhalten (J. Med. Chem. 10, 1015 (1967)).

1-Benzyl-3-(N-ethyl-N-methylamino)-pyrrolidin kann durch Lithiumaluminiumhydrid-Reduktion von 3-Acetylamino-1-benzyl-pyrrolidin-2,5-dion, gefolgt von der Methylierung mit Formaldehyd, erhalten werden (J. Med. Chem. 20, 801 (1977)).

3-Amino-1-benzylpyrrolidin wurde durch Hydrierung von 1-Benzyl-3(hydroximino)pyrrolidin erhalten (Japan. Kokai 7826161). 3-Amino-1-benzylpyrrolidin wurde außerdem durch Umsetzung von 1-Benzyl-3-phthalimidopyrrolidin mit Hydrazin erhalten (J. Med. Chem. 11, 1034 (1968). Beiden Verfahren zur Herstellung von 3-Amino-1-benzylpyrrolidin ist gemeinsam, daß die benötigten Edukte in einer vielstufigen verlustreichen Synthese hergestellt werden müssen (siehe z.B. US 3 691 197, J. Org. Chem. 30, 740 (1965)).

Weiterhin ist bekannt, daß N-Phenyl-maleinimid mit Methylamin zu 3-Methylamino-1-phenyl-pyrrolidin-2,5-dion umgesetzt und dieses mit Lithiumaluminiumhydrid zu 3-Methylamino-1-phenyl-pyrrolidin reduziert werden kann (J. Med. Chem. 10, 1015 (1967)).

3-Dimethylamino-pyrrolidin wurde durch Addition von Dimethylamin an N-Benzylmaleinimid zu 1-Benzyl-3-dimethylamino-pyrrolidin-2,5-dion und anschließende Reduktion mit Lithiumaluminiumhydrid und katalytische Hydrierung erhalten (Eur.J.Med.Chem. 13, 479 (1978)).

3-Amino-pyrrolidine mit identischen Substituenten an beiden Stickstoffatomen werden bei der Umsetzung von 1,2,4-trisubstituierten Butanen mit Aminen erhalten (EP 0218 249).

Über die Addition von Stickstoffnucleophilen an 1-Benzyl-$\Delta^3$-pyrrolin-2,5-dione zur Herstellung von 3-Amino-1-benzyl-pyrrolidinen ist nichts bekannt.

Es wurde nun gefunden, daß man 3-Amino-1-benzyl-pyrrolidine der Formel (I)

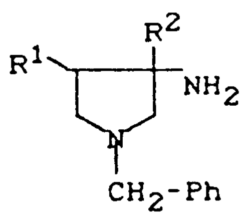

(I)

wobei

$R^1$ und $R^2$, gleich oder verschieden, jeweils H oder $C_1$-$C_6$-Alkyl, insbesondere aber H bedeuten,

erhält, wenn man 1-Benzyl-$\Delta^3$-pyrrolin-2,5-dione der Formel (II) mit Stickstoffnucleophilen der Formel (III) zu gegebenenfalls substituierten 3-Amino-1-benzyl-pyrrolidin-2,5-dionen der Formel (IV) umsetzt,

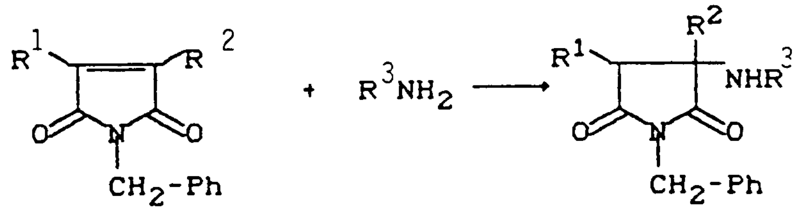

(II) (III) (IV)

wobei

$R^3$ entweder H, Benzyl, Naphthylmethyl oder ein Substituent Ph-CHR$^4$ bedeutet mit $R^4$ = $C_1$-$C_6$-Alkyl oder Phenyl und

$R^1$ und $R^2$ die oben angegebene Bedeutung haben,

anschließend für den Fall $R^3 \neq H$ die Schutzgruppe $R^3$ abspaltet zu 3-Amino-1-benzyl-pyrrolidin-2,5-dionen (IVa)

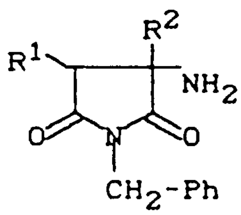

(IVa)

wobei
$R^1$ und $R^2$ die oben angegebene Bedeutung haben,
und durch vollständige Reduktion der Carbonylgruppen (IVa) in 3-Amino-1-benzyl-pyrrolidine (I) überführt

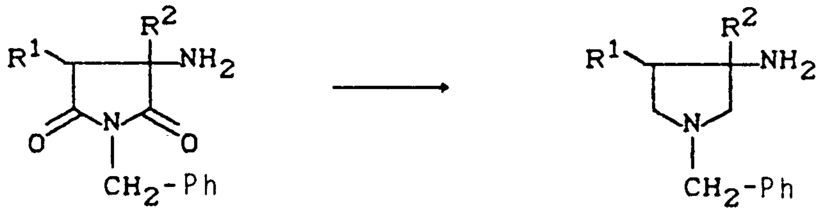

(IVa)

wobei $R^1$ und $R^2$ die oben angegebene Bedeutung haben.

Außerdem wurde gefunden, daß man 3-(Aralkylamino)-1-benzyl-pyrrolidine der Formel erhält, wenn man 3-(Aralkylamino)-1-benzyl-pyrrolidin-2,5-dione der Formel (IVb) reduziert,

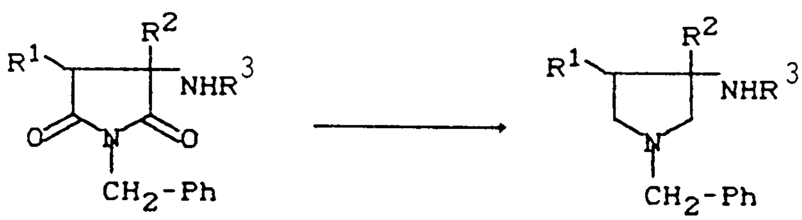

(IVb) (V)

wobei
$R^1$, $R^2$ und $R^3$ die oben angegebene Bedeutung haben,
$R^3$ aber $\neq H$ ist.

Der erste Reaktionsschritt des erfindungsgemäßen Verfahrens, die Addition von Stickstoffnucleophilen der Formel (III) an 1-Benzyl-$\Delta^3$-pyrrolin-2,5-dione der Formel (II), wird durchgeführt, indem man die Reaktanden in einem Lösungsmittel oder auch ohne Lösungsmittel bei Temperaturen von -40 °C bis +200 °C miteinander umsetzt. Die Wahl des Lösungsmittels und der Reaktionstemperatur richtet sich dabei nach der Reaktivität der Edukte und nach ihrer Neigung, Nebenreaktionen oder Folgereaktionen mit dem Produkt einzugehen.

Als Lösungsmittel können in reiner Form oder als Gemisch verwendet werden: aliphatische und aromatische Kohlenwasserstoffe, wie z.B. Ligroin, Cyclohexan, Toluol, Xylol und Tetralin, offenkettige und cyclische Ether wie z.B. Diethylether, Methyl-tert.-butylether, Di-n-butylether, Dimethoxyethan, Diethylenglykoldimethylether, Diethylenglykoldiethylether, Tetrahydrofuran, Dioxan und Anisol, chlorierte Kohlenwasserstoffe wie Chlorbenzol, Chloroform und 1,2-Dichlotethan, Ester wie Ameisensäureethylester, Essigsäureethylester, Glykolmonomethyletheracetat und Essigsäure-n-butylester sowie dipolar aprotische Lösungsmittel wie Dimethylformamid, Dimethylsulfoxid, N-Methylpyrrolidinon, N-Methylcaprolactam, Tetramethylharnstoff und Sulfolan.

Bevorzugt sind aromatische Kohlenwasserstoffe sowie cyclische und offenkettige Ether und deren Gemische.

Im Falle von $R^3 \neq H$ ist es zweckmäßig, die Reaktanden im stöchiometrischen oder annähernd stöchiometrischen Verhältnis miteinander umzusetzen, während im Falle von $R^3 = H$ auch ein Überschuß des Ammoniaks angezeigt sein kann.

Die Reaktionsführung kann entweder so erfolgen, daß das Stickstoffnucleophil der Formel (III) zu 1-Benzyl-$\Delta^3$-pyrrolin-2,5-dion der Formel (II) gegeben wird, wobei beide Reaktionspartner in Substanz oder in Lösung vorliegen können, oder die Verbindungen der Formel (II) zu einer vorgelegten Verbindung der Formel (III) gegeben wird. Es ist aber auch möglich, beide Reaktionspartner simultan in einem Reaktionsgefäß zu dosieren. Die Reaktion kann bei Normaldruck, vermindertem Druck und auch bei erhöhtem Druck durchgeführt werden.

Die Art der Reaktionsführung richtet sich u.a. nach der Reaktivität des Stickstoffnucleophils (III), insbesondere bei der Verwendung von Ammoniak ($R^3 \neq H$) ist es sinnvoll, überschüssigen Ammoniak vorzulegen, um Folgereaktionen der Produkte des Typs (IVa) mit Edukten der Formel (II) in Form einer weiteren Addition zu verringern.

Die erhaltenen substituierten 3-Amino-1-benzyl-pyrrolidin-2,5-dione der Formel (IV) sind teilweise bekannt, sie wurden jedoch bisher auf andere Weise hergestellt (siehe z.B. J. Am. Chem. Soc. 70, 3778 (1948), J. Pharm. Sci. 70, 192 (1981)).

Zur Herstellung der 3-Amino-1-benzyl-pyrrolidin-2,5-dione (IVa) muß, falls bei der Additionsreaktion nicht Ammoniak als Stickstoffnucleophil der Formel (III) ($R^3 = H$) verwendet wurde, die Schutzgruppe am 3-Amino-Substituenten der Pyrrolidin-2,5-dione (IV) abgespalten werden.

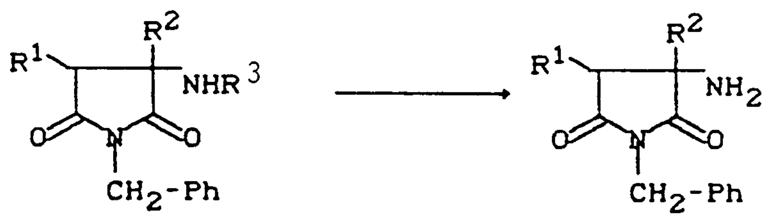

(IV) (IVa)

Die Abspaltung der Schutzgruppe wird durch katalytische Hydrierung erreicht. Als Lösungsmittel können Alkohole, cyclische und offenkettige Ether, Carbonsäuren, alkoholische Salzsäure, Alkylaromaten und deren Gemische untereinander oder mit Wasser verwendet werden. Als Katalysatoren dienen Palladium,, Palladium auf Trägern, wie z.B. Aktivkohle, Platin, Raney-Nickel oder Raney-Cobalt bei Temperaturen von 0 bis 200 °C und Wasserstoffdrucken von 1 bis 300 bar. Bevorzugt ist die Verwendung von Alkoholen oder Ethern als Lösungsmittel und Palladium auf Aktivkohle als Katalysator.

Die Reduktion der Carbonylgruppen von (IVa) zu 3-Amino-1-benzylpyrrolidinen (I) sowie von 3-(Aralkylamino)-1-benzyl-pyrrolidin-2,5-dionen der Formel (IVb) zu 3-(Aralkylamino)-1-benzyl-pyrrolidinen der Formel (V) kann durch katalytische Hydrierung oder durch Umsetzung mit Hydriden oder komplexen Hydriden der Elemente der 3. Hauptgruppe erfolgen. Als Hydrid kommt bevorzugt Borhydrid, auch in Form von Komplexen mit Lewis-Säuren oder Lewis-Basen zum Einsatz, als komplexe Hydride bevorzugt Natrium-bis-(2-methoxyethoxy)aluminiumhydrid, Lithiumaluminiumhydrid sowie Natriumborhydrid, mit oder ohne Zusatz von Lewis-Säuren, wie z.B. Titantetrachlorid, Zinntetrachlorid, Aluminiumtrichlorid, Bortrifluorid, Eisentrichlorid und Antimonpentachlorid.

Die Reduktionen werden in inerten organischen Lösunsmitteln wie cyclische oder offenkettige Ether, wie z.B. Diethylether, Methyl-tert.-butylether, Di-n-butylether, Dimethoxyethan, Diethylenglykoldimethylether, Diethylenglykoldiethylether, Tetrahydrofuran oder Dioxan oder Alkylaromaten, wie z.B. Toluol, Xylol und Tetralin oder deren Gemische durchgeführt bei Temperaturen von 0 bis 200 °C. Zum Erreichen höherer Reaktionstemperaturen und niedrigerer Reaktionszeiten kann die Reduktion mit Hydriden oder komplexen Hydriden auch bei Drucken >1 bar durchgeführt werden.

In einer besonderen Ausführungsform des Verfahrens werden die einzelnen Zwischenstufen nicht isoliert, sondern als Reaktionslösung jeweils in der folgenden Stufe eingesetzt. Diese Vereinfachung hat im allgemeinen keine oder nur geringe chemisch bedingte Ausbeuteverluste zur Folge, und die Aufarbeitungsverluste werden auf die letzte Reaktionsstufe beschränkt. Als Lösungsmittel kommen dann nur solche in Frage, die für alle Reaktionsstufen geeignet sind, wie cyclische und offenkettige Ether und Alkylaromaten.

Es ist als außerordentlich überraschend zu bezeichnen, daß gemäß des erfindungsgemäßen Verfahrens 3-Amino-1-benzylpyrrolidine der Formel (I) in so guten Ausbeuten hergestellt werden können. Insbesondere war es nicht zu erwarten, daß die Reaktionsfolge ohne Isolierung der Zwischenstufen in hohen Ausbeuten durchgeführt werden kann. Im Hinblick auf den Stand der Technik mußte man erwarten, daß Neben- und Folgereaktionen zu Ausbeuteverlusten führen würden und Verunreinigungen der Zwischenstufen, die eine Isolierung erforderlich machen, mit sich bringen würden.

So war beispielsweise für die Addition von Benzylamin an 1-Benzyl-$\Delta^3$-pyrrolin-2,5-dion zu erwarten, daß die Ringöffnung als Neben- oder Folgereaktion zu Ausbeuteverlusten und zur Verunreinigung des Produktes führen würde, da bekannt ist, daß bei der Additon von primären Aminen an 1-Benzyl-$\Delta^3$-pyrrolin-2,5-dion schon bei Raumtemperatur eine doppelte Addition unter Ringöffnung zu Amino-bernsteinsäurediamiden erfolgen kann (Revue Roumaine de Chimie 15, 253 (1970)).

Weiterhin war für die Addition der Stickstoffnucleophile der Formel (III) an 1-Benzyl-$\Delta^3$-pyrrolin-2,5-dione der Formel (II) zu erwarten, daß das Additionsprodukt der Formel (IV) mit der Ausgangsverbindung der Formel (II) eine weitere Additionsreaktion eingeht (vgl. J. March, Advanced Organic Chemistry, S. 689, 3. Ausgabe, John Wiley & Sons, New York 1985).

Auch für die Abspaltung der Schutzgruppe $R^3$ der Formel (IV) zu 3-Amino-1-benzyl-pyrrolidin-2,5-dionen (IVa) war mit Schwierigkeiten zu rechnen, insbesondere, wenn die Reaktionslösung gleich für den folgenden Reaktionsschritt eingesetzt werden sollte. So konnte z.B. bei der Debenzylierung von 1-Benzyl-3-benzylamino-pyrrolidin-2,5-dion zu 3-Amino-1-benzyl-pyrrolidin-2,5-dion eine gleichzeitige Abspaltung der 1-Benzylgruppe nicht ausgeschlossen werden, da zuweilen auch die N-Benzylgruppen in Säureamiden unter relativ milden Bedingungen hydrogenolytisch abgespalten werden können (J. Org. Chem. 27, 3606 (1962)). Außerdem wurde selbst bei der Abspaltung einer Carbobenzoxyschutzgruppe - die stets wesentlich leichter erfolgt als die Hydrogenolyse eines N-Benzylrestes - eine konkurrierende Ringöffnung bei 3-Benzyl-8-carbobenzoxy-3,8-diazobicyclo[3.2.1]-2,4-dion beobachtet (J. Org. Chem. 26, 2747 (1961)). Es war zu erwarten, daß diese Nebenreaktionen besonders dann Schwierigkeiten bereiten würden, wenn wegen der Einschränkungen der Lösungsmittel-Auswahl bei einer Reaktionsfolge ohne Isolierung der Zwischenstufen besonders drastische Reaktionsbedingungen bei der katalytischen Debenzylierung angewandt werden müßten.

Denn häufig wird die hydrogenolytische Spaltung von Benzylaminen in protischen Lösungsmitteln in Gegenwart einer Säure durchgeführt, um die Vergiftung des Katalysators durch freie Amine zu verhindern (M. Freifelder, "Practical Catalytic Hydrogenation", S. 39 und S. 414, Wiley-Interscience New York 1971).

Das erfindungsgemäße Verfahren weist eine Reihe von Vorteilen auf. Als Ausgangsstoffe verwendet es 1-Benzyl-$\Delta^3$-pyrrolin-2,5-dione, welche z.B. in einfacher Weise aus Maleinsäureanhydrid oder Citraconsäureanhydrid und Benzylamin erhalten werden können (J. Org. Chem. 25, 1012-1015 (1960)). Die Reaktionsschritte des erfindungsgemäßen Verfahrens ergeben überwiegend sehr gute Ausbeuten. Darüber hinaus ist es häufig möglich, ohne Nachteile in Kauf nehmen zu müssen, mehrere Reaktionsstufen im gleichen Lösungsmittel ohne Aufarbeitung der Zwischenstufen durchzuführen.

So kann beispielsweise 3-Amino-1-benzyl-pyrrolidin erfindungsgemäß hergestellt werden, indem 1-Benzyl-$\Delta^3$-pyrrolidin-2,5-dion mit Benzylamin umgesetzt wird, anschließend durch katalytische Hydrierung selektiv zu 3-Amino-1-benzyl-pyrrolidin-2,5-dion debenzyliert und dieses mit Lithiumaluminiumhydrid oder Natriumborhyd/Lewis-Säure reduziert wird.

Erfindungsgemäß erhält man 1-Benzyl-3-benzyl-amino-pyrrolidin, indem man 1-Benzyl-$\Delta^3$-pyrrolidin-2,5-dion mit Benzylamin zu 1-Benzyl-3-benzyl-amino-pyrrolidin-2,5-dion umsetzt und dieses mit Lithiumaluminiumhydrid oder Natriumborhydrid/Lewis-Säure reduziert.

Diese Verfahren können ohne Isolierung der Zwischenstufen in einem zyklischen oder offenkettigen Ether durchgeführt werden.

Verwendet man 1-Benzyl-$\Delta^3$-pyrrolin-2,5-dion als Ausgangsstoff, Benzylamin als Stickstoffnucleophil, Lithiumaluminiumhydrid als Reduktionsmittel und Palladium auf Aktivkohle für die Debenzylierung, so kann der Reaktionsablauf durch das folgende Formelschema wiedergegeben werden:

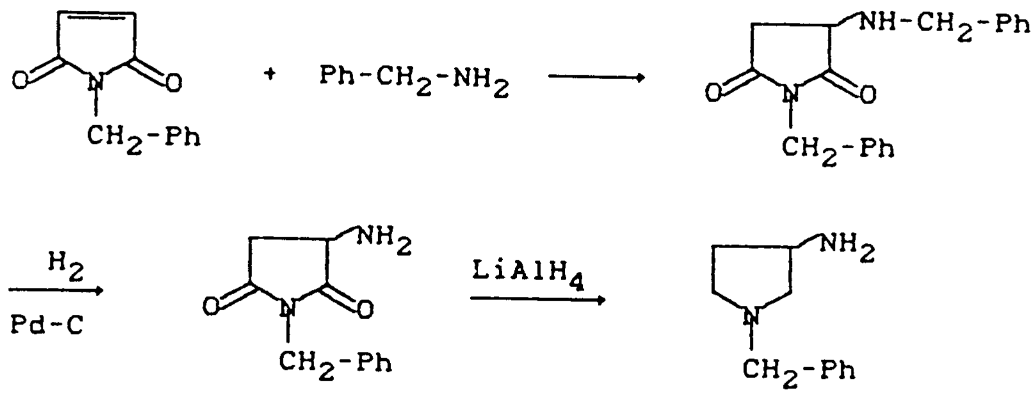


Die Verbindungen der Formel (I) dienen als Zwischenprodukte zur Herstellung von 3-Aminopyrrolidin oder von 3-tert.-Butyloxycarbonylamino-pyrrolidin, welche zur Synthese antibakterieller Chinoloncarbonsäurederivate benötigt werden (vgl. EP 153 163, EP 218 249, DE 3 601 517 und US 4 382 937).

## Beispiele

### Beispiel 1

1-Benzyl-3-benzylamino-pyrrolidin-2,5-dion

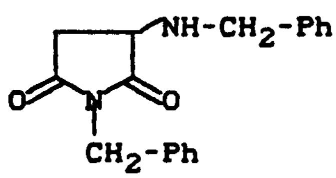

37,4 g (0,2 Mol) 1-Benzyl-$\Delta^3$-pyrrolin-2,5-dion wird in 100 ml Tetrahydrofuran vorgelegt und bei 20 °C 21,4 g (0,2 Mol) Benzylamin zugetropft, worauf die Temperatur auf 40 °C ansteigt. Es wird noch 1 Stunde bei dieser Temperatur nachgerührt, dann wird das Lösungsmittel am Rotationsverdampfer bis 80 °C Badtemperatur bei 6 mbar entfernt. Es bleiben 59,3 g eines Festkörpers zurück mit einem gaschromatographisch bestimmten Gehalt von 96 %. Ausbeute 97 % der Theorie, Schmelzpunkt: 60 bis 63 °C.

Beispiel 2

1-Benzyl-3-benzylamino-3-methyl-pyrrolidin-2,5-dion

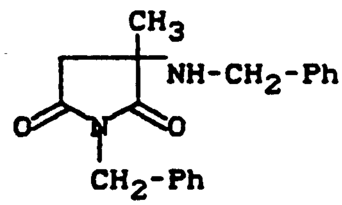

120,6 g (0,6 Mol) Citraconsäure-N-benzylimid und 65,2 g (0,6 Mol) Benzylamin werden in 600 ml Tetrahydrofuran 16 Stunden am Rückfluß gekocht. Dann wird das Lösungsmittel abdestilliert und der Rückstand auf Kieselgel mit Essigsäureethylester-Petrolether 1:1 chromatographiert. Es werden 76,2 g eines Öls erhalten, welches nach gaschromatographischer Analyse 93 %ig ist (Ausbeute 38 % der Theorie).
$^1$H-NMR ($CDCl_3$): 1,41 (s, 3H);
(200 MHz, δH in ppm) 1,85 (s, 1H);
2,69 (2d, 2H, J = 18 Hz)
3,52 (2d, 2H, J = 11 Hz)
4,68 (s, 2H);
7,2-7,4 (m, 10H).

Beispiel 3

3-Amino-1-benzyl-pyrrolidin-2,5-dion

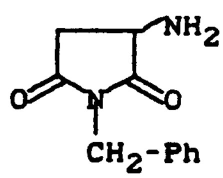

Methode A:

56,5 g (0,184 Mol) 95 %iges 1-Benzyl-3-benzylamino-pyrrolidin-2,5-dion wird in 700 ml Ethanol gelöst und mit 2 g Palladium auf Aktivkohle (5 %) 4 Stunden bei 70 ° C und 60 bar Wasserstoffdruck hydriert. Dann wird der Katalysator abfiltriert, das Filtrat eingeengt und Rückstand bei 50 ° C/0,1 mbar getrocknet. Es werden 36,2 g Produkt mit einem gaschromatographisch bestimmten Gehalt von 94 % erhalten, was einer Ausbeute von 91 % der Theorie entspricht. Schmelzpunkt 74 ° bis 77 ° C.

Methode B:

1.500 ml Tetrahydrofuran werden vorgelegt und bei 0 ° C soviel Ammoniak eingeleitet, bis an einem aufgesetzten Trockeneiskühler Ammoniak kondensiert. Unter Ammoniakeinleiten werden dann 374 g (2 Mol) 1-Benzyl-$\Delta^3$-pyrrolin-2,5-dion als Lösung in 500 ml Tetrahydrofuran zugetropft und anschließend 3 Stunden bei 0 ° C nachgerührt. Die Lösung wird am Rotationsverdampfer eingeengt und der Rückstand in der doppelten Gewichtsmenge Essigsäureethylester aufgenommen. Die nach eintägigem Stehen bei 0 ° C erhaltenen Kristalle werden abgesaugt und die Mutterlauge auf 2 kg Kieselgel aufgebracht. Die Elution erfolgt zunächst mit Essigsäureethylester, dann mit Essigsäureethylester-Ethanol 1:1. Die Fraktionen, die nur das Produkt mit einem RF-Wert von 0,43 (Kieselgel, Essigsäureethylster-Ethanol 1:1) enthalten, werden zusammen eingeengt. Es werden 185,7 g 3-Amino-1-benzyl-pyrrolidin-2,5-dion, Schmelzpunkt 73 bis 75 ° C, erhalten.

Beispiel 4

3-Amino-1-benzyl-pyrrolidin

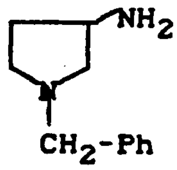

Methode A:

34 g (0,9 Mol) Lithiumaluminiumhydrid werden unter Stickstoff in 500 ml wasserfreiem Tetrahydrofuran vorgelegt und 80,8 g (0,4 Mol) 3-Amino-1-benzyl-pyrrolidin-2,5-dion als Lösung in 400 ml Tetrahydrofuran zugetropft. Dann wird 20 Stunden unter Rückflußkühlung gekocht. Zu dem Ansatz werden nacheinander 34 g Wasser in 120 ml Tetrahydrofuran, 34 g 10 %ige Kalilauge sowie 136 g Wasser getropft. Der Festkörper wird abgesaugt, mit THF gewaschen, in 250 ml Tetrahydrofuran ausgekocht und erneut abgesaugt. Die vereinigten Filtrate werden am Rotationaverdampfer eingeengt und der Rückstand unter vermindertem Druck destilliert.
Siedepunkt: 78 bis 85 ° C/0,1 bis 0,2 mbar,
Ausbeute: 50,5 g, nach gaschromatographischer Analyse 92 %iges Produkt (≙ 66 % der Theorie).

Methode B:

Aus 1-Benzyl-$\Delta^3$-pyrrolin-2,5-dion, Ammoniak und Lithiumaluminiumhydrid ohne Isolierung der Zwischenstufen
300 ml Tetrahydrofuran werden vorgelegt und bei 0 ° C soviel Ammoniak eingeleitet, bis das Gas an einem aufgesetzten Trockeneiskühler kondensiert. Unter gleichzeitigem Ammoniak-Einleiten wird dann eine Lösung von 74,9 g (0,4 Mol) 1-Benzyl-$\Delta^3$-pyrrolin-2,5-dion in 100 ml THF zugetropft und dann 3 Stunden bei 0 ° C nachgerührt. Aus dem Ansatz werden 200 ml Tetrahydrofuran abdestilliert und der Rückstand unter Stickstoff zu 34 g (0,9 Mol) Lithiumaluminiumhydrid in 700 ml Tetrahydrofuran getropft. Dann wird 10 Stunden unter Rückflußkühlung gekocht, nacheinander mit 34 g Wasser in 120 ml Tetrahydrofuran, 34 g 10 %ige Natronlauge und 102 g Wasser hydrolysiert, der Niederschlag abgesaugt und mit Tetrahydrofuran gewaschen, das Filtrat am Rotationsverdampfer eingeengt und der Rückstand destilliert. Es werden 21 g Produkt mit einem gaschromatographisch bestimmten Gehalt von 94 % erhalten, was einer Ausbeute von 28 % der Theorie entspricht.

Methode C:

Aus 1-Benzyl-$\Delta^3$-pyrrolin-2,5-dion und Benzylamin, gefolgt von der katalytischen Hydrierung und der Reduktion, ohne Isolierung der Zwischenstufen.

374 g (2 Mol) 1-Benzyl-$\Delta^3$-pyrrolin-2,5-dion werden in 2 l Tetrahydrofuran gelöst und bei 0 bis 10 ° C 214 g (2 Mol) Benzylamin zugegeben. Es wird noch 1 Stunde bei Raumtemperatur nachgerührt. Die Hydrierung erfolgt mit 30 g Palladium 5 %ig auf Aktivkohle bei 90 bis 100 ° C und 100 bar Wasserstoffdruck und ist nach 4 Stunden beendet.

Der Katalysator wird abfiltriert und mit 100 ml Tetrahydrofuran gewaschen. 664 g dar so erhaltenen Lösung (≙ 28 % der erhaltenen 2.370 g) werden zu einer Lösung von 47,6 g (1,25 Mol) Lithiumaluminiumhydrid in 700 ml wasserfreiem Tetrahydrofuran in einem 3 1-Autoklaven gepumpt und anschließend 10 Stunden auf 100 ° C erhitzt. Dann werden nacheinander unter Ableiten des entstehenden Wasserstoffs 420 ml Tetrahydrofuran, 48 ml Wasser in 168 ml Tetrahydrofuran, 48 g 10 %ige Kalilauge sowie 143 ml Wasser zugepumpt, der Ansatz über eine Drucknutsche filtriert, der Filterkuchen mit THF gewaschen und mit 500 ml THF ausgekocht und erneut abgesaugt und die vereinigten Filtrate eingeengt. Die Destillation ergibt 70,2 g 3-Amino-1-benzyl-pyrrolidin-2,5-dion mit einem Gehalt von 96,1 %, Siedpunkt: 75 bis 80 ° C/0,13 bis 0,2 mbar. Dies entspricht einer Ausbeute von 68,4 %, bezogen auf 1-Benzyl-$\Delta^3$-pyrrolin-2,5-dion, oder einer durchschnittlichen Ausbeute von 88,1 % pro Reaktionsstufe.

Beispiel 5

1-Benzyl-3-benzylamino-pyrrolidin

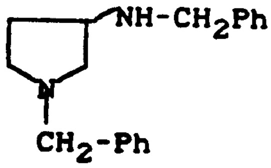

Methode A:

1.000 g (5,34 Mol) 1-Benzyl-$\Delta^3$-pyrrolin-2,5-dion werden in 2,5 1 Tetrahydrofuran gelöst und unter Rühren bei 10 bis 20 ° C 572 g (5,34 Mol) Benzylamin zugesetzt. Nach 24 Stunden Stehen bei Raumtemperatur werden von der erhaltenen Lösung (3,848 g Gesamtgewicht) 360 g entnommen und zu einer Lösung von 38 g (1 Mol) Lithiumaluminiumhydrid in 380 ml absolutiertem Tetrahydrofuran getropft. Es wird 20 Stunden unter Rückflußkühlung gekocht, dann nacheinander 500 ml Tetrahydrofuran, 38 g Wasser in 120 ml Tetrahydrofuran, 38 g 10 %ige Kalilauge und 114 g Wasser zugesetzt. Die Salze werden abgesaugt, mit Tetrahydrofuran gewaschen, das Filtrat eingeengt und der Rückstand destilliert.
Siedepunkt: 151 bis 164 ° C/0,19 mbar, Ausbeute: 111,3 g, nach gaschromatographischer Analyse 99,6 %ig, entsprechend einer Ausbeute von 84 % der Theorie, bezogen auf 1-Benzyl-$\Delta^3$-pyrrolin-2,5-dion.

Methode B:

450 g der bei Methode A aus Benzylamin und 1-Benzyl-$\Delta^3$-pyrrolin-2,5-dion gewonnenen Lösung werden mit 47,6 g (1,25 Mol) Lithiumaluminiumhydrid im Autoklaven innerhalb von 10 Stunden bei 100 ° C reduziert. Die Aufarbeitung erfolgt wie bei Methode A aus Beispiel 4. Es werden 144,1 g (86 % der Theorie) 1-Benzyl-3-benzylamino-pyrrolidin erhalten.

Methode C:

56,7 g (0,4 Mol) Bortrifluorid-Diethylether-Addukt wird unter Stickstoff zu einer Lösung von 28,3 g (0,75 Mol) Natriumborhydrid in 700 ml Diethylenglykoldimethylether getropft. Dazu werden bei 0 bis 5 ° C 216 g (0,3 Mol) der nach Methode A erhaltenen Lösung von 1-Benzyl-3-benzylamino-pyrrolidin-2,5-dion in Tetrahydrofuran getropft. Dann wird 15 Stunden bei 100 ° C nachgerührt. Dann werden 223 ml 6 n Salzsäure zugetropft und auf 100 ° C erhitzt, bis die Gasentwicklung beendet ist. Die Mischung wird nach dem Abkühlen weiter mit Wasser verdünnt und mit Natronlauge pH 11 eingestellt. Das gebildete Öl wird im Toluol aufgenommen, die Toluol-Phase mit Natriumsulfat getrocknet und das Lösungsmittel bei 80 ° C Badtemperatur/5 mbar entfernt. Bei der Hochvakuumdestillation werden 60,7 g Produkt, Sdp. 165-170 ° C/0,21 mbar, mit einem Gehalt von 97,6 % erhalten, was einer Ausbeute von 74 % der Theorie entspricht.

Beispiel 6

1-Benzyl-3-benzylamino-3-methyl-pyrrolidin

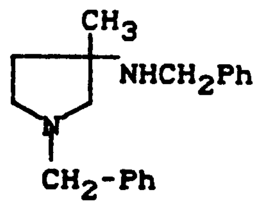

51 g 93 %iges 1-Benzyl-3-benzylamino-3-methyl-pyrrolidin-2,5-dion (0,154 Mol) wird in 330 ml Dimethoxyethan mit 12,4 g (0,33 Mol) Lithiumaluminiumhydrid reduziert. Die Aufarbeitung erfolgt wie bei A in Beispiel 5.
Siedepunkt: 141 bis 143 °C/0,13 mbar. Ausbeute: 30,1 g, nach gaschromatographischer Analyse 98,5 %ig (≙ 69 % der Theorie).

Beispiel 7

3-Amino-1-benzyl-pyrrolidin

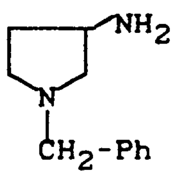

4151 g (22,2 Mol) 1-Benzyl-$\Delta^3$-pyrrolin-2,5-dion werden in 10,4 l Tetrahydrofuran (Wassergehalt <0,1 %) vorgelegt und 2375 g (22,2 Mol) Benzylamin bei 10 - 30 °C zugetropft. Über Nacht wird bei Raumtemperatur nachgerührt, dann wird mit 10 l Tetrahydrofuran (Wassergehalt <0,1 %) verdünnt und bei 90 - 100 °C und 90 - 100 bar über 300 g 5 %igem Palladium auf Aktivkohle hydriert. Zur Vervollständigung der Debenzylierung werden am Ende der Wasserstoffaufnahme nochmals 100 g Katalysator zugesetzt und unter den oben beschriebenen Bedingungen weiterhydriert. Dann wird der Katalysator abfiltriert und mit 1,5 l Tetrahydrofuran (Wassergehalt <0,1 %) gewaschen.

Das Filtrat wird (bis auf eine Probe von 270 g ≅ 0,26 Mol) bei 20 °C zu 18,5 kg (48,7 Mol) vorgelegter 10 %iger Lithiumaluminiumhydridlösung in Tetrahydrofuran sowie 7 l Tetrahydrofuran (Wassergehalt < 0,1 %) in einen 130 l Rührautoklaven gepumpt und die Leitung mit 2 l Tetrahydrofuran (Wassergehalt <0,1 %) gespült. Dann wird 10 Stunden auf 100 °C erhitzt, der Wasserstoff nach dem Abkühlen entspannt und nacheinander bei 20-30 °C 16,8 l Tetrahydrofuran, 1850 ml Wasser in 5,5 l Tetrahydrofuran, 1850 ml 10 %ige Kalilauge sowie 5,5 l Wasser zugepumpt. Der Ansatz wird bis zur Druckkonstanz gerührt, der Wasserstoff entspannt und die Reaktionsmischung filtriert. Der Filterkuchen wird mit Tetrahydrofuran ausgekocht, erneut filtriert und die vereinigten Filtrate am Rotationsverdampfer eingeengt. Aus dem Rückstand (3840 g) werden durch Hochvakuumdestillation ohne Kolonne und ohne zu fraktionieren 3336 g Rohdestillat erhalten, aus dem durch Destillation über eine 60 cm Vigreuxkolonne 3131 g Produkt (Sdp. 94-96 °C/0,18 mbar) mit einem gaschromatographisch bestimmten Gehalt von 96,7 % erhalten werden. Dies entspricht einer Ausbeute von 78,3 %, bezogen auf 1-Benzyl-$\Delta^3$-pyrrolin-2,5-dion, oder einer durchschnittlichen Ausbeute von 92,2 % pro Reaktionsstufe.

**Patentansprüche**

1. Verfahren zur Herstellung von 3-Amino-1-benzyl-pyrrolidinen der Formel (I)

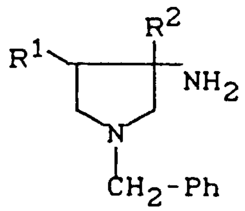

(I)

worin
$R^1$ und $R^2$ gleich oder verschieden sind und jeweils H oder $C_1$-$C_6$-Alkyl bedeuten,
dadurch gekennzeichnet, daß man 1-Benzyl-$\Delta^3$-pyrrolin-2,5-dione der Formel (II)

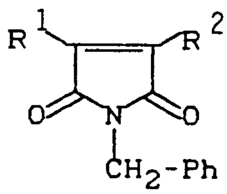

(II)

mit Stickstoffnucleophilen der Formel (III)

$R^3NH_2$ (III)

zu gegebenenfalls substituierten 3-Amino-1-benzylpyrrolidin-2,5-dionen der Formel (IV)

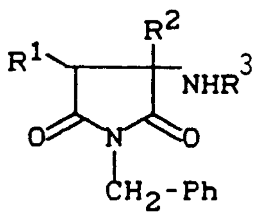

(IV)

umsetzt,
wobei
$R^3$ für H, Benzyl, Naphthylmethyl oder für einen Substituenten Phenyl-$CHR^4$ mit $R^4$ = $C_1$-$C_6$-Alkyl oder Phenyl steht
und $R^1$ und $R^2$ die oben angegebene Bedeutung haben,
anschließend für den Fall $R^3 \neq H$ die Schutzgruppe $R^3$ abspaltet zu 3-Amino-1-benzyl-pyrrolidin-2,5-dionen der Formel (IVa)

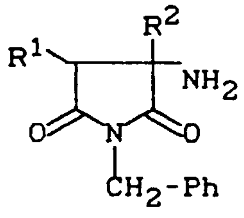

(IVa)

und durch vollständige Reduktion der Carbonylgruppen in die 3-Amino-1-benzyl-pyrrolidine der Formel (I) überführt.

**2.** Verfahren zur Herstellung von Verbindungen der Formel (V)

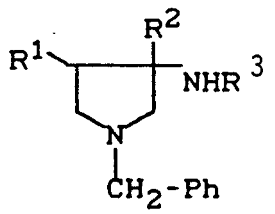

(V)

in der
$R^1$, $R^2$ und $R^3$ die in Anspruch 1 angegebene Bedeutung haben, wobei $R^3$ aber ≠ H ist,
dadurch gekennzeichnet, daß man Verbindungen der Formel (IVb)

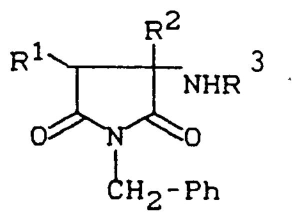

katalytisch oder durch Umsetzung mit Hydriden oder komplexen Hydriden der 3. Hauptgruppe des Periodensystems der Elemente hydriert.

**3.** Verfahren nach den Ansprüchen 1 und 2, dadurch gekennzeichnet, daß man als Reduktionsmittel Borhydrid, Natrium-bis-(2-methoxy)aluminiumhydrid, Lithiumaluminiumhydrid oder Natriumborhydrid mit oder ohne Zusatz von Lewis-Säuren verwendet.

**4.** Verfahren nach den Ansprüchen 1 bis 3, dadurch gekennzeichnet, daß man die Reduktion in Ethern oder Alkylaromaten oder deren Gemische durchführt.

**5.** Verfahren nach den Ansprüchen 1 bis 4, dadurch gekennzeichnet, daß die Substituenten $R^1$ und $R^2$ Wasserstoff bedeuten.

**6.** Verfahren nach Anspruch 5, dadurch gekennzeichnet, daß die Reaktionsfolge ohne Isolierung der Zwischenstufen erfolgt.

**7.** Verfahren zur Herstellung von 3-Amino-1-benzyl-pyrrolidin, dadurch gekennzeichnet, daß man 1-Benzyl-$\Delta^3$-pyrrolin-2,5-dion mit Benzylamin umsetzt, durch katalytische Hydrierung selektiv zu 3-Amino-1-benzyl-pyrrolidin-2,5-dion debenzyliert und dieses mit Lithiumaluminiumhydrid oder Natriumborhydrid/Lewis-Säure reduziert.

**8.** Verfahren nach Anspruch 7, dadurch gekennzeichnet, daß die Reaktionsfolge ohne Isolierung der Zwischenstufen in einem cyclischen oder offenkettigen Ether erfolgt, und daß als Hydrierkatalysator Palladium auf Aktivkohle eingesetzt wird.

**9.** Verfahren zur Herstellung von 1-Benzyl-3-benzylamino-pyrrolidin, dadurch gekennzeichnet, daß man 1-Benzyl-$\Delta^3$-pyrrolin-2,5-dion mit Benzylamin zu 1-Benzyl-3-benzylamino-pyrrolidin-2,5-dion umsetzt und dieses mit Lithiumaluminiumhydrid oder Natriumborhydrid/Lewis-Säure reduziert.

**10.** Verfahren nach Anspruch 9, dadurch gekennzeichnet, daß die Reaktionsfolge ohne Isolierung der Zwischenstufe in einem cyclischen oder offenkettigen Ether erfolgt.

**Claims**

1. Process for preparing 3-amino-1-benzylpyrrolidines of the formula (I)

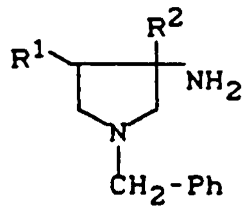

(I)

in which
$R^1$ and $R^2$ are identical or different and each denote H or $C_1$-$C_6$-alkyl,
characterised in that 1-benzyl-$\Delta^3$-pyrroline-2,5-diones of the formula (II)

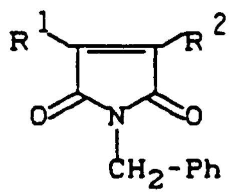

(II)

are reacted with nitrogen nucleophiles of the formula (III)

$R^3NH_2$ (III)

to give optionally substituted 3-amino-1-benzylpyrrolidine-2,5-diones of the formula (IV)

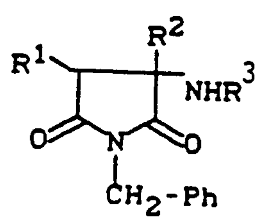

(IV)

where
$R^3$ represents H, benzyl, naphthylmethyl or a substituent phenyl-$CHR^4$ where $R^4$ is $C_1$-$C_6$-all or phenyl and
$R^1$ and $R^2$ have the abovementioned meaning, and, in the case of $R^3 \neq H$, the protecting group $R^3$ is subsequently cleaved off to give 3-amino-1-benzylpyrrolidine-2,5-diones of the formula (IVa)

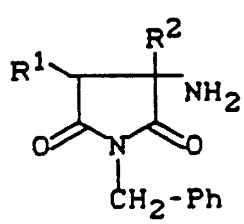

(IVa)

which are converted to the 3-amino-1-benzyl-pyrrolidines of the formula (I) by complete reduction of the carbonyl groups.

**2.** Process for preparing compounds of the formula (V)

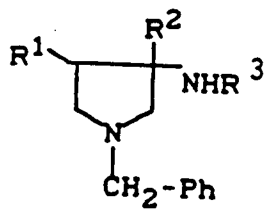

(V)

in which
$R^1$, $R^2$ and $R^3$ have the meaning specified in Claim 1, but $R^3 \neq H$,
characterised in that compounds of the formula (IVb)

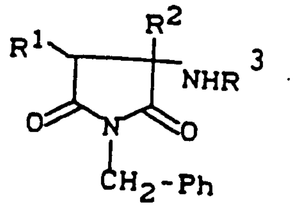

(IVb)

are hydrogenated catalytically or by reaction with hydrides or complex hydrides of the 3rd main group of the periodic table of the elements.

**3.** Process according to Claims 1 and 2, characterised in that the reducing agent used is boron hydride, sodium bis-(2-methoxy)aluminium hydride, lithium aluminium hydride or sodium borohydride with or without addition of Lewis acids.

**4.** Process according to Claims 1 to 3, characterised in that the reduction is carried out in ethers or alkyl-aromatics or mixtures thereof.

**5.** Process according to Claims 1 to 4, characterised in that the substituents $R^1$ and $R^2$ denote hydrogen.

**6.** Process according to Claim 5, characterised in that the reaction sequence is carried out without isolation of the intermediates.

**7.** Process for preparing 3-amino-1-benzyl-pyrrolidine, characterised in that 1-benzyl-$\Delta^3$-pyrroline-2,5-dione is reacted with benzylamine, the product is debenzylated by catalytic hydrogenation to give selectively 3-amino-1-benzylpyrrolidine-2,5-dione which is reduced using lithium aluminium hydride or sodium borohydride/Lewis acid.

**8.** Process according to Claim 7, characterised in that the reaction sequence is carried out in a cyclic or open-chain ether without isolation of the intermediates, and that palladium on activated charcoal is used as the hydrogenation catalyst.

**9.** Process for preparing 1-benzyl-3-benzylamino-pyrrolidine, characterised in that 1-benzyl-$\Delta^3$-pyrroline-2,5-dione is reacted with benzylamine to give 1-benzyl-3-benzylamino-pyrrolidine-2,5-dione which is reduced using lithium aluminium hydride or sodium borohydride/Lewis acid.

**10.** Process according to Claim 9, characterised in that the reaction sequence is carried in a cyclic or open-chain ether without isolation of the intermediate.

**Revendications**

1. Procédé pour la fabrication de 3-amino-1-benzyl-pyrrolidines de formule (I)

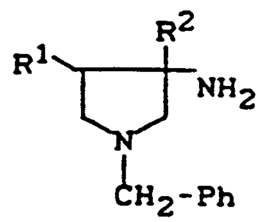

(I)

dans laquelle
$R^1$ et $R^2$ sont identiques ou différents et représentent chaque fois H ou un groupe alkyle en $C_1$-$C_6$, caractérisé en ce que l'on fait réagir des 1-benzyl-$\Delta^3$-pyrroline-2,5-diones de formule (II)

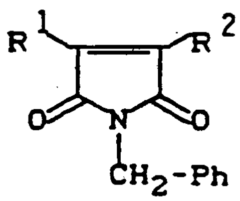

(II)

avec des nucléophiles azotés de formule (III)

$R^3NH_2$ (III)

pour former des 3-amino-1-benzylpyrrolidine-2,5-diones éventuellement substituées de formule (IV)

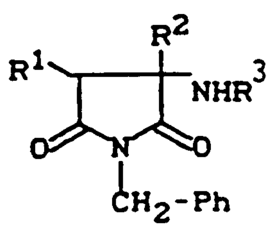

dans laquelle
$R^3$ représente l'hydrogène, un groupe benzyle, naphtylméthyle ou un substituant phényl-$CHR^4$, $R^4$ étant alkyle en $C_1$-$C_6$ ou phényle
et $R^1$ et $R^2$ possèdent les significations décrites ci-dessus,
que, ensuite, dans le cas où $R^3$ est différent de H, on scinde le groupe protecteur $R^3$ en 3-amino-1-benzylpyrrolidine-2,5-diones de formule (IVa)

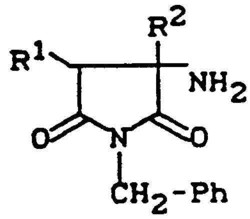

(IVa)

et qu'on, transforme par réduction complète des groupes carbonyle, en 3-amino-1-benzyl-pyrrolidines de formule (I).

2. Procédé pour la fabrication de composés de formule (V)

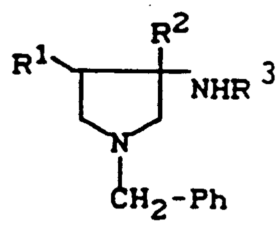

(V)

dans laquelle
$R^1$, $R_2$ et $R^3$ possèdent les significations décrites dans la revendication 1, mais où $R^3$ est différent de H, caractérisé en ce que l'on hydrogène des composés de formule (IVb)

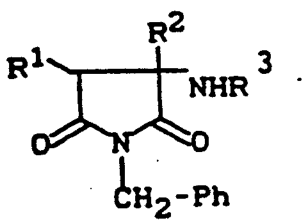

avec catalysation ou en les faisant réagir avec des hydrures ou avec des hydrures complexes du troisième groupe principal du système de classification périodique des éléments.

3. Procédé selon les revendications 1 et 2, caractérisés en ce que l'on emploie, comme agent réducteur, de l'hydrure de bore, de l'hydrure de sodium-bis-(2-méthoxy)aluminium, de l'hydrure de lithium-aluminium ou de l'hydrure de sodium-bore avec ou sans addition d'acides de Lewis.

4. Procédé selon les revendications 1 à 3, caractérisé en ce que l'on effectue la réduction dans des éthers ou des hydrocarbures aromatiques alkylés ou leurs mélanges.

5. Procédé selon les revendications 1 à 4, caractérisé en ce que les substituants $R^1$ et $R^2$ signifient l'hydrogène.

6. Procédé selon la revendication 5, caractérisé en ce que la réaction est effectuée sans isolation des stades intermédiaires.

7. Procédé pour la fabrication de 3-amino-1-benzyl-pyrrolidine, caractérisé en ce que l'on fait réagir de la 1-benzyl-$\Delta^3$-pyrroline-2,5-dione avec de la benzylamine, en ce qu'on effectue une débenzylation sélective par hydrogénation catalytique pour obtenir de la 3-amino-1-benzylpyrrolidine-2,5-dione, et qu'on réduit celle-ci avec de l'hydrure de lithium-aluminium ou de l'hydrure de sodium-bore/acide de Lewis.

**8.** Procédé selon la revendication 7, caractérisé en ce que la réaction est effectuée sans isolation des stades intermédiaires dans un éther cyclique ou à chaîne ouverte, et que l'on emploie comme catalyseur d'hydrogénation du palladium sur du charbon actif.

**9.** Procédé pour la fabrication de 1-benzyl-3-benzylaminopyrrolidines, caractérisé en ce que l'on fait réagir de la 1-benzyl-$\Delta^3$-pyrroline-2,5-dione avec de la benzylamine pour former de la 1-benzyl-3-benzylamino-pyrrolidine-2,5-dione et qu'on réduit celleci avec de l'hydrure de lithium-aluminium ou de l'hydrure de sodium-bore/acide de Lewis.

**10.** Procédé selon la revendication 9, caractérisé en ce que la réaction est effectuée sans isolation des stades intermédiaires dans un éther cyclique ou à chaîne ouverte.